# EUROPÄISCHE PATENTANMELDUNG

(11) **EP 4 722 609 A1**
(43) Veröffentlichungstag der Anmeldung: **08.04.2026**
(21) Anmeldenummer: 25205784.9
(22) Anmeldetag: 30.09.2025
(51) Int. Cl.: F24F 11/36, G01N 33/00

(54) **SCHWERGASDETEKTIONSVORRICHTUNG**

(30) Priorität: 04.10.2024 DE 202024105743 U
(71) Anmelder: IT Inventor GmbH, 48465 Schüttorf / Samern (DE)
(72) Erfinder: Satar, Mehmet, 48531 Nordhorn (DE)
(74) Vertreter: Werner & ten Brink PAe PartGes mbB Bentheim

(57) **Zusammenfassung**

Angegeben wird eine Schwergasdetektionsvorrichtung (10) mit einem Gehäuse (12) und mit einer Luftansaugvorrichtung (18), einer Sensorik (20) und einer Auffangvorrichtung (22) im Innern des Gehäuses (12) sowie mit zumindest einen Lufteinlass (14) in das Innere des Gehäuses (12), wobei sich die Sensorik (20) in einem Strömungsweg vom Lufteinlass (14) zur Luftansaugvorrichtung (18) befindet und in der Auffangvorrichtung (22) platziert ist.

## Beschreibung

Die hier vorgeschlagene Neuerung betrifft das technische Gebiet der sogenannten HLK-Systeme oder HLKK-Systeme (HLK = Heizungs-, Lüftungs- und Klimatechnik; HLKK = Heizungs-, Lüftungs-, Klima- und Kältetechnik). Andere Abkürzungen sind ebenfalls geläufig: HLS (Heizung, Lüftung, Sanitär), TGA (Technische Gebäudeausrüstung), HKL (Heizung, Klima/Kälte, Lüftung), HLS (Heizung, Lüftung, Sanitär), SHK (Sanitär, Heizung, Klima) sowie HVAC (Heating, Ventilation, Air Conditioning). In der hier vorgelegten Beschreibung wird zusammenfassend der Begriff Kältemaschine verwendet. Eine Kältemaschine ist eine HLK-Anlage oder ein HLK-System oder ein Teil eines HLK-Systems. Konkret betrifft die hier vorgeschlagene Neuerung das technische Gebiet der Detektion von aus einer Kältemaschine im Fehlerfall austretendem Schwergas.

Kältemaschinen werden in an sich bekannter Art und Weise mit einem sogenannten Kältemittel betrieben. Als Kältemittel kommen mehr und mehr Propan oder Kohlenstoffdioxid (CO₂) oder Erdgas (CH₄), zusammenfassend als Schwergas bezeichnet, zur Anwendung. Im Fehlerfall austretendes Gas, insbesondere Schwergas, muss sicher erkannt (sensiert/detektiert) werden. Dafür ist eine entsprechende Sensorik notwendig. Als Sensor einer solchen Sensorik zur Detektion von austretendem Gas, insbesondere Schwergas, kommt ein grundsätzlich bekannter sogenannter NDIRSensor (Nichtdispersiver Infrarotsensor) in Betracht.

Eine Aufgabe der hier vorgeschlagenen Neuerung besteht entsprechend darin, eine Detektionsvorrichtung zur Detektion von aus einer Kältemaschine austretendem Kältemittel anzugeben, insbesondere eine Detektionsvorrichtung, die zur Detektion von austretendem Gas, insbesondere Schwergas, zum Beispiel Propan und dergleichen geeignet ist.

Diese Aufgabe wird erfindungsgemäß mittels einer Detektionsvorrichtung, insbesondere einer zur Detektion von Schwergas und dergleichen bestimmten Detektionsvorrichtung (Schwergasdetektionsvorrichtung), mit den Merkmalen des Anspruchs 1 gelöst. Die Detektionsvorrichtung umfasst ein Gehäuse und eine Sensorik im Innern des Gehäuses. Die Besonderheit besteht darin, dass das Gehäuse einen Lufteinlass in das Innere des Gehäuses in Form einer oder mehrerer Öffnungen im Gehäuse sowie eine Luftansaugvorrichtung im Innern des Gehäuses aufweist, wobei mittels der Luftansaugvorrichtung beim Betrieb der Detektionsvorrichtung Luft aus der Umgebung der Detektionsvorrichtung (Umgebungsluft) in das Innere des Gehäuses gesaugt wird und wobei sich die Sensorik in einem Strömungsweg vom Lufteinlass zur Luftansaugvorrichtung befindet, nämlich in einem Strömungsweg eines Luftstroms im Innern des Gehäuses und durch das Gehäuse beim Betrieb der Luftansaugvorrichtung. Mittels der Luftansaugvorrichtung wird Umgebungsluft in das Innere des Gehäuses gesaugt/gefördert. Wenn in der Umgebung der Detektionsvorrichtung Schwergas ausgetreten ist, sammelt sich dies am Boden und wird mit der angesaugten Umgebungsluft in das Innere der ebenfalls am Boden platzierten Detektionsvorrichtung mitgerissen. Somit gelangt das Schwergas auch in das Innere des Gehäuses der Detektionsvorrichtung und kann dort mittels der dort befindlichen Sensorik erkannt (sensiert/detektiert) werden. Aufgrund des Ansaugens der Umgebungsluft und des Mitreißens von ggf. ausgetretenem Schwergas ist die Erkennung eines Schwergasaustritts bereits zu einem frühen Zeitpunkt möglich, wenn die tatsächlich ausgetretene Schwergasmenge noch gering ist und eine Detektion mittels einer lediglich knapp über Bodenniveau platzierten Sensorik noch nicht möglich ist.

Es hat sich jüngst gezeigt, dass auch Gase wie Methan zumindest zum Teil zum Boden absinken und sich dort ("in Fetzen") sammeln. Auch solche Gase sollen für die Auslegung der hier vorgelegten Beschreibung als von der Bezeichnung Schwergas umfasst gelten.

Vorteilhafte Ausgestaltungen der hier vorgeschlagenen Detektionsvorrichtung sind Gegenstand der weiteren Ansprüche. Dabei verwendete Rückbeziehungen innerhalb der Ansprüche weisen auf die weitere Ausbildung der Detektionsvorrichtung gemäß der Definition des in Bezug genommenen Anspruchs hin, nämlich die weitere Ausbildung durch die Merkmale des jeweiligen abhängigen Anspruchs. Solche weiteren Ausbildungen sind nicht als ein Verzicht auf die Erzielung eines selbständigen, gegenständlichen Schutzes für die Merkmale oder Merkmalskombinationen eines abhängigen Anspruchs zu verstehen. Des Weiteren ist im Hinblick auf eine Auslegung der Ansprüche sowie der Beschreibung bei einer näheren Konkretisierung eines Merkmals in einem abhängigen Anspruch davon auszugehen, dass eine derartige Beschränkung in dem oder jedem vorangehenden Anspruch sowie einer allgemeineren Ausführungsform der Detektionsvorrichtung nicht vorhanden ist. Jede Bezugnahme in der Beschreibung auf Aspekte abhängiger Ansprüche ist demnach auch ohne speziellen Hinweis ausdrücklich als Beschreibung optionaler Merkmale zu lesen.

Bei einer vorteilhaften Ausführungsform der hier vorgeschlagenen Detektionsvorrichtung ist vorgesehen, dass diese im Innern ihres Gehäuses eine Auffangvorrichtung aufweist und dass die Sensorik in der Auffangvorrichtung platziert ist. Mittels der Luftansaugvorrichtung beim Ansaugen von Umgebungsluft ggf. mitgerissenes Schwergas sinkt auch im Innern des Gehäuses der Detektionsvorrichtung zu Boden. Die Auffangvorrichtung fängt im Innern des Gehäuses absinkendes Schwergas auf und bewirkt, dass sich das absinkende Schwergas in der Auffangvorrichtung sammelt. Dort kann das gesammelte Schwergas mittels der in der Auffangvorrichtung platzierten Sensorik gut und sicher erkannt werden und zwar auch dann, wenn in der Umgebung der Detektionsvorrichtung erst geringe Schwergasmengen ausgetreten sind.

Vorteilhaft ist bezüglich der Auffangvorrichtung vorgesehen, dass diese eine Wanne und einen umlaufenden Rand umfasst. Mit dem Rand liegt die Auffangvorrichtung an den Innenwänden des Gehäuses der Detektionsvorrichtung an; die Auffangvorrichtung wird mit dem Rand formschlüssig vom Gehäuse der Detektionsvorrichtung aufgenommen. Die Wanne ist der Bereich, in dem sich im Innern des Gehäuses absinkendes Schwergas sammelt. Die Bodenfläche der Wanne ist kleiner als die Grundfläche des Gehäuses der Detektionsvorrichtung. Bei sich in der Wanne sammelndem Schwergas steigt dort der Pegel also schneller als bei auf den Boden des Gehäuses absinkendem Schwergas. Die Sensorik reicht in die Wanne hinein, so dass sich dort sammelndes Schwergas schnell, gut und sicher erkannt werden kann.

Weiter vorteilhaft ist bezüglich der Auffangvorrichtung vorgesehen, dass diese zumindest eine Öffnung aufweist, durch welche mittels der Luftansaugvorrichtung in der Umgebung der Detektionsvorrichtung und durch den zumindest einen Lufteinlass angesaugte Umgebungsluft und mit dieser ggf. mitgerissenes Schwergas in die Auffangvorrichtung und zu der dort befindlichen Sensorik gelangt. Dann führt der Strömungsweg der angesaugten Umgebungsluft durch die Auffangvorrichtung hindurch und der resultierende Luftstrom strömt durch die Auffangvorrichtung hindurch. Ggf. mitgerissenes Schwergas gelangt damit so gut wie möglich in den Bereich der Sensorik.

Die mit der Anmeldung eingereichten Patentansprüche sind Formulierungsvorschläge ohne Präjudiz für die Erzielung weitergehenden Patentschutzes. Da speziell die Merkmale der abhängigen Ansprüche im Hinblick auf den Stand der Technik am Prioritätstag eigene und unabhängige Erfindungen bilden können, behält die Anmelderin sich vor, diese oder noch weitere, bisher nur in der Beschreibung und/oder Zeichnung offenbarte Merkmalskombinationen zum Gegenstand unabhängiger Ansprüche oder Teilungserklärungen zu machen. Sie können weiterhin auch selbständige Erfindungen enthalten, die eine von den Gegenständen der jeweils in Bezug genommenen Ansprüche unabhängige Gestaltung aufweisen.

Nachfolgend wird als Ausführungsbeispiel eine Ausführungsform der hier vorgeschlagenen Detektionsvorrichtung anhand der Zeichnung näher erläutert. Einander entsprechende Gegenstände oder Elemente sind in allen Figuren mit den gleichen Bezugszeichen versehen.

Das Ausführungsbeispiel ist nicht als Einschränkung der Erfindung zu verstehen. Vielmehr sind im Rahmen der vorliegenden Offenbarung durchaus auch Ergänzungen und Modifikationen möglich, insbesondere solche, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen oder Verfahrensschritten für die Fachperson im Hinblick auf die Lösung der Aufgabe entnehmbar sind und durch kombinierbare Merkmale zu einem neuen Gegenstand oder zu neuen Verfahrensschritten bzw. Verfahrensschrittfolgen führen.

Es zeigen
- Fig. 1 und Fig. 2: isometrische Ansichten einer Schwergasdetektionsvorrichtung (Detektionsvorrichtung),
- Fig. 3: eine Frontansicht der Detektionsvorrichtung aus Fig. 1 und Fig 2 sowie
- Fig. 4, Fig. 5 und Fig. 6: einen Schnitt durch die Detektionsvorrichtung gemäß der in Fig. 3 gezeigten Schnittlinie.

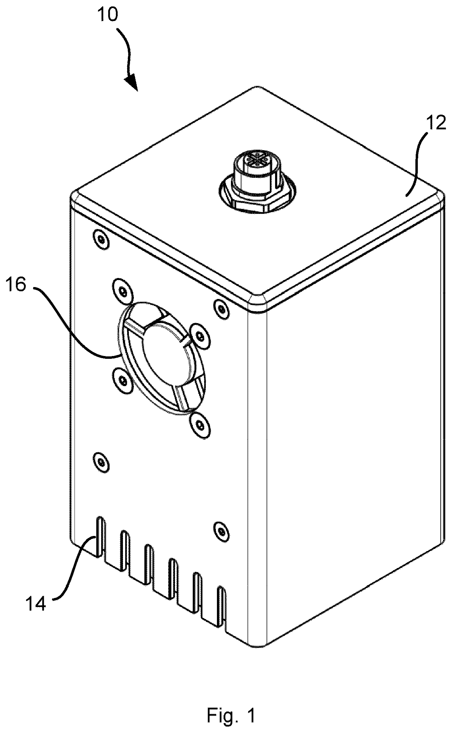

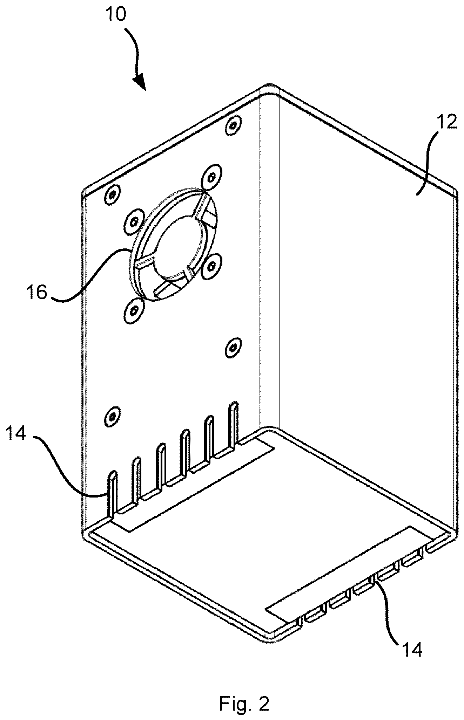

Die Darstellungen in Figur 1 und Figur 2 zeigen aus verschiedenen Ansichten eine beispielhafte Ausführungsform einer Detektionsvorrichtung 10 der hier vorgeschlagenen Art. Die Detektionsvorrichtung 10 umfasst ersichtlich ein Gehäuse 12. Bei dem Gehäuse 12 handelt es sich bei der gezeigten Ausführungsform um ein kubisches Gehäuse 12. Ebenfalls in Betracht kommen ein zylindrisches Gehäuse 12 oder ein Gehäuse 12 mit einer polygonalen Grundfläche. Jedenfalls weist das Gehäuse 12 eine Grundfläche und eine Höhe auf. Bevorzugt ist die Höhe größer als jede Dimension der Grundfläche, also bei dem gezeigten kubischen Gehäuse jeweils größer als deren Breite und Tiefe.

Das Gehäuse 12 weist zumindest einen Lufteinlass 14 auf. Bei der gezeigten Ausführungsform fungieren als Lufteinlass 14 mehrere von einer Unterkante des Gehäuses 12 ausgehende und parallel zur Hochachse des Gehäuses 12 orientierte Schlitze (nur einer bezeichnet). In der Darstellung in Figur 2 - die bei der gewählten Perspektive auch die Unterseite der Detektionsvorrichtung 10 zeigt - ist erkennbar, dass die Detektionsvorrichtung 10 bei der gezeigten Ausführungsform die als Lufteinlass 14 fungierenden Schlitze in zwei einander gegenüberliegenden Seitenflächen aufweist. Der oder jeder Lufteinlass 14 fungiert als Ansaugkanal der Detektionsvorrichtung 10. Eventuelle mehrere benachbarte Schlitze oder dergleichen, wie bei der gezeigten Ausführungsform, bilden zusammen einen Ansaugkanal. Die gezeigte Ausführungsform weist also in den beiden mit Schlitzen versehenen, gegenüberliegenden Seitenflächen jeweils einen Ansaugkanal auf. Stege zwischen den Schlitzen sichern die Öffnung des jeweiligen Ansaugkanals gegen den Eintritt von störenden Gegenständen.

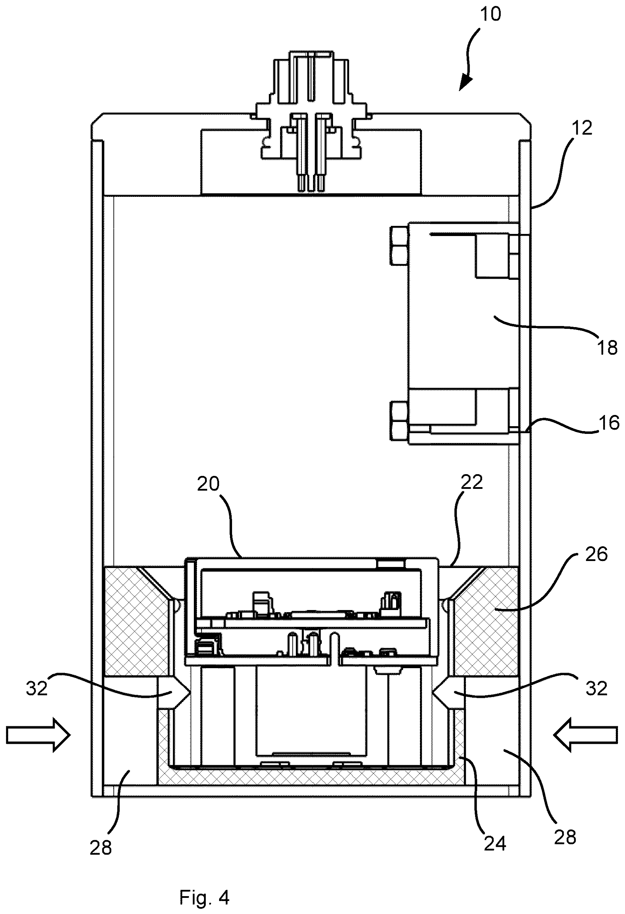

Das Gehäuse 12 weist des Weiteren zumindest einen Luftauslass 16 auf (die Bezugslinie geht zum Rand der hier kreisförmigen und als Luftauslass 16 fungierenden Öffnung im Gehäuse 12). Hinter dem Luftauslass 16 erkennt man die Flügel eines Lüfterrads. Das Lüfterrad gehört zu einem grundsätzlich an sich bekannten Lüfter und der Lüfter ist ein Beispiel für eine bei der Detektionsvorrichtung 10 vorgesehene Luftansaugvorrichtung 18 (Fig. 4).

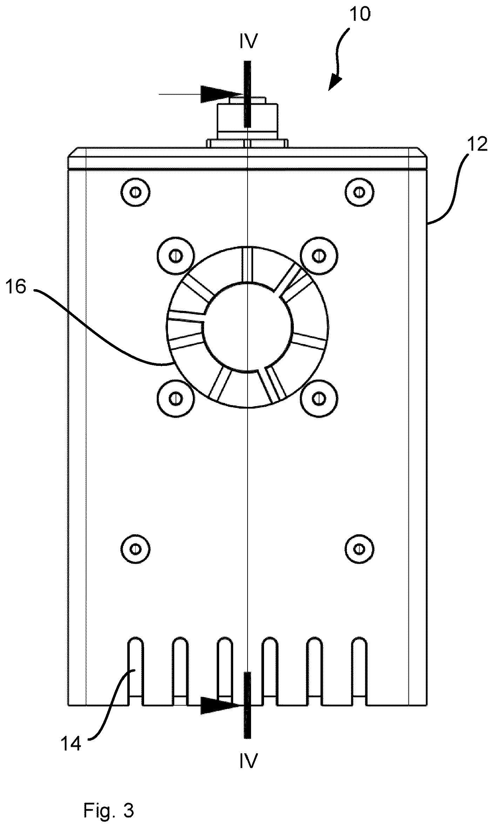

Die Darstellung in Figur 3 zeigt eine Frontansicht der Detektionsvorrichtung 10 aus Figur 1 und Figur 2. Man erkennt wiederum gut das Lüfterrad des Lüfters (Luftansaugvorrichtung 18) hinter dem Luftauslass 16 sowie die als Lufteinlass 14 fungierenden Schlitze. Die anderen auf der Oberfläche des Gehäuses 12 der Detektionsvorrichtung 10 gezeigten Einzelheiten sind Teile oder Orte von Verbindungselementen (Schrauben oder dergleichen).

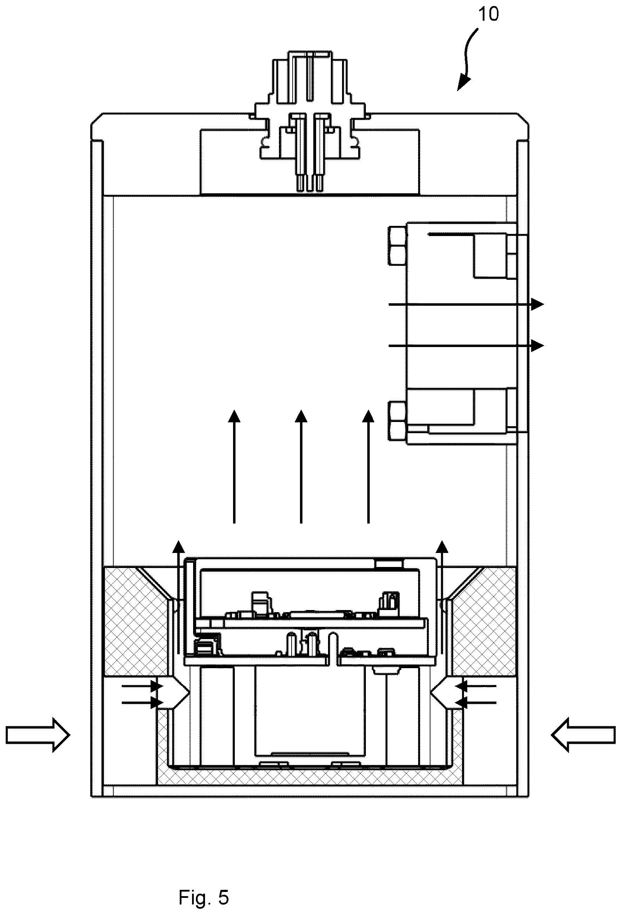

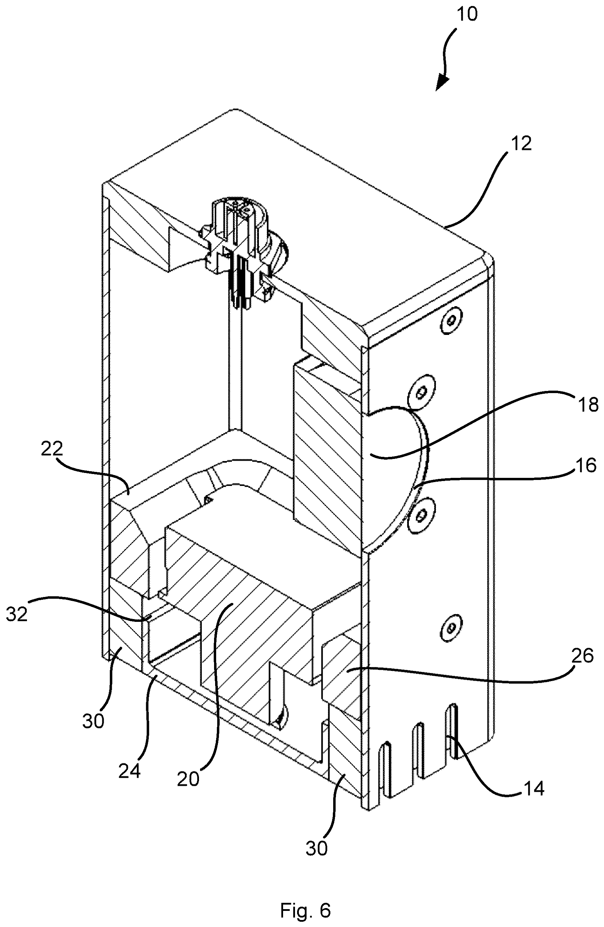

Die Darstellungen in Figur 4 und Figur 5 zeigen einen Schnitt durch die Detektionsvorrichtung 10 aus Figur 1, Figur 2 und Figur 3 entlang der in Figur 3 eingezeichneten Schnittlinie IV-IV. Die Darstellung in Figur 6 zeigt die Detektionsvorrichtung 10 entlang derselben Schnittebene wie in Figur 4 oder Figur 5, aber aus einer anderen Perspektive. In der Darstellung in Figur 6 sind die Luftansaugvorrichtung 18 und eine Sensorik 20 schematisch vereinfacht nur noch als Volumenmodelle gezeigt.

Die beiden in Figur 4 eingezeichneten, seitlich in Richtung auf das Gehäuse 12 weisenden Blockpfeile veranschaulichen die Richtung einer beim Betrieb der Luftansaugvorrichtung 18 resultierenden Luftströmung in das Gehäuse 12 durch den Lufteinlass 14. Im Innern des Gehäuses 12 befinden sich die Luftansaugvorrichtung 18 (bei der gezeigten Ausführungsform der als Luftansaugvorrichtung 18 fungierende Lüfter) und eine Sensorik 20. Die Sensorik 20 ist ihrerseits im Innern des Gehäuses 12 in einer Auffangvorrichtung 22 platziert. Die Sensorik 20 hat im weitesten Sinne eine T-Form mit zum Beispiel einer Elektronik im horizontalen Abschnitt und dem zumindest einen Sensor im vertikalen Abschnitt, nämlich bevorzugt im unteren Bereich des vertikalen Abschnitts. Die Sensorik 20 befindet sich im Innern des Gehäuses 12 über (in Richtung der Hochachse des Gehäuses 12) dem Lufteinlass 14 und somit über dem oder jedem Ansaugkanal und der Luftauslass 16 sowie die Luftansaugvorrichtung 18 befinden sich im Innern des Gehäuses 12 über der Sensorik 20. Als Material für die Auffangvorrichtung 22 kommt Kunststoff in Betracht. Die Auffangvorrichtung 22 ist insoweit zum Beispiel als Kunststoffspritzgussteil ausgeführt.

Die Auffangvorrichtung 22 umfasst - insbesondere einstückig - eine Wanne 24 sowie einen nach oben (in Richtung der Hochachse des Gehäuses 12) an die Wanne 24 anschließenden Rand 26. Mit dem Rand 26 liegt die Auffangvorrichtung 22 bevorzugt allseitig an den Innenoberflächen der Seitenwände des Gehäuses 12 an. Das Gehäuse 12 nimmt die Auffangvorrichtung 22 dann mit ihrem Rand 26/aufgrund ihres Rands 26 formschlüssig auf. Insgesamt ist die Auffangvorrichtung 22 ein Einsatz, der von dem Gehäuse 12/im Gehäuse 12 bevorzugt formschlüssig aufgenommen wird. Der Rand 26 umgibt zudem die Sensorik 20, wobei zwischen der Sensorik 20 und dem Rand 26 zumindest auf einzelnen Seiten der Sensorik 20, insbesondere allseitig, ein Abstand bleibt.

Während der Rand 26 der Auffangvorrichtung 22 allseitig bevorzugt unmittelbar an den jeweils angrenzenden Innenoberflächen des Gehäuses 12 anliegt, insbesondere dichtend anliegt, bleibt zwischen der Wanne 24 und jeder Gehäuseseitenwand mit einem Lufteinlass 14 ein Zwischenraum. Der Zwischenraum wird als Filtertasche 28 bezeichnet. Jede Filtertasche 28 ist zur Aufnahme jeweils eines Filterelements 30 (Figur 6) oder allgemein einer Schmutzpartikelrückhaltevorrichtung bestimmt. Jede Filtertasche 28 nimmt ein jeweiliges Filterelement 30 formschlüssig auf. Ein Filterelement 30 ist dafür in Bezug auf die Dimension der Filtertasche 28 leicht übermassig, nämlich in Bezug auf die Länge und Breite der Filtertasche 28 (quer zur Hochachse des Gehäuses 12 gemessen) leicht übermassig.

Die Sensorik 20 ist in der Auffangvorrichtung 22 so angeordnet, dass der davon umfasste Sensor oder zumindest ein davon umfasster Sensor in Richtung auf den Boden der Wanne 24 ausgerichtet ist. Die Sensorik 20 ist insoweit in der Auffangvorrichtung 22 gewissermaßen kopfüber eingebaut. Die Auffangvorrichtung 22 umgibt die Sensorik 20 und die Sensorik 20 befindet sich in der Auffangvorrichtung 22 oder zumindest im Wesentlichen in der Auffangvorrichtung 22, d.h. die Sensorik 20 oder zumindest ein Teil der Sensorik 20 nimmt ein Teil des von der Auffangvorrichtung 22 umgebenen Volumens ein.

Mittels der Luftansaugvorrichtung 18 wird beim Betrieb der Detektionsvorrichtung 10 Umgebungsluft in das Innere des Gehäuses 12 gesaugt und somit ein Luftstrom durch das Innere des Gehäuses 12 erzeugt. Der Luftstrom tritt über den oder jeden Lufteinlass 14 in das Gehäuse 12 ein und tritt über den oder jeden Luftauslass 16 wieder aus. Die Luftansaugvorrichtung 18 befindet sich in Bezug auf den resultierenden Luftstrom durch das Gehäuse 12 stromaufwärts des oder jedes Luftauslasses 16, die Auffangvorrichtung 22 und die dortige Sensorik 20 befinden sich wiederum stromaufwärts der Luftansaugvorrichtung 18 und der oder jeder Lufteinlass 14 befindet sich stromaufwärts der Sensorik 20/Auffangvorrichtung 22. Vom Lufteinlass 14 aus betrachtet, befinden sich die Auffangvorrichtung 22 und die Sensorik 20 stromabwärts des Lufteinlasses 14, die Luftansaugvorrichtung 18 stromabwärts der Auffangvorrichtung 22 und der Sensorik 20 und schließlich der Luftauslass 16 stromabwärts der Luftansaugvorrichtung 18.

Der Luftstrom durch das Gehäuse 12 strömt auch durch die Auffangvorrichtung 22 hindurch. Dafür weist die Auffangvorrichtung 22 im Bereich ihrer Wanne 24 zumindest eine Öffnung 32 im Bereich ihrer Seitenwandung auf und ist ansonsten im Bereich ihres umlaufenden Rands 26 nach oben (in Richtung der Hochachse des Gehäuses 12; in Richtung auf die Luftansaugvorrichtung 18) trog-/schalenartig offen. Als Beispiele für die zumindest eine Öffnung 32 in der Wandung der Wanne 24 sind in den Darstellungen in Figur 4, Figur 5 und Figur 6 horizontale Schlitze gezeigt, nämlich jeweils ein horizontaler Schlitz in jeder einer Seitenwand des Gehäuses 12 mit Lufteinlass 14 benachbarten Seitenwand der Wanne 24. In den Darstellungen in Figur 4 und Figur 5 erscheinen diese Schlitze als auf der Seite liegendes "Haussymbol" (Konturen eines Hauses mit einem Satteldach). Diese Geometrie resultiert in der Darstellung daraus, dass der Schlitz in Radien beim Übergang der geschlitzten Wandung in die anschließende Wandung mündet.

Der beim Betrieb der Luftansaugvorrichtung 18 resultierende Luftstrom im Gehäuse 12 ist in der Darstellung in Figur 5 mittels einzelner Pfeile veranschaulicht. Die Darstellung in Figur 5 ist - im Interesse der besseren Übersichtlichkeit - eine Wiederholung der Darstellung in Figur 4 ohne Bezugsziffern, aber mit diesen Pfeilen. Es wird zunächst auf die Pfeile im Bereich der Öffnungen 32 hingewiesen. Hier tritt der Luftstrom in die Auffangvorrichtung 22 ein. Sodann wird auf die Pfeile beidseitig der Sensorik 20 verwiesen. Hier verlässt der Luftstrom den Bereich der Auffangvorrichtung 22. Die Platzierung der Pfeile neben der Sensorik 20 ist allein der zweidimensionalen Schnittdarstellung geschuldet. Der Luftstrom wird sich auch vor und hinter der Sensorik 20 einstellen. Sodann sind Pfeile in der Mitte des Gehäuses 12 und im Bereich der Luftansaugvorrichtung 18 gezeigt. Hier gelangt der Luftstrom zur Luftansaugvorrichtung 18 und verlässt durch die Luftansaugvorrichtung 18 und den anschließenden Luftauslass 16 das Gehäuse 12.

Das Ansaugen von Umgebungsluft (Luft aus der Umgebung der Detektionsvorrichtung 10) zum Erhalt des beschriebenen Luftstroms durch das Gehäuse 12 hat den Zweck, eventuelles Schwergas, zum Beispiel Propan oder dergleichen, in der Umgebung der Detektionsvorrichtung 10 aufzunehmen und zur Sensorik 20 der Detektionsvorrichtung 10 zu leiten. Schwergas sinkt nach unten und breitet sich auf dem Boden aus. Damit Schwergas mit einer entsprechenden Sensorik sensiert werden kann, ist grundsätzlich eine gewisse Schichtdicke und damit eine nicht unerhebliche Menge von ausgetretenem Schwergas erforderlich. Es ist aber nicht tolerierbar, eine Austrittsmenge abzuwarten, bis eine detektierbare Schichtdicke erreicht ist. Daher wird bei der hier vorgeschlagenen Detektionsvorrichtung 10 kontinuierlich oder zu vorgegebenen oder vorgebbaren Zeitpunkten während einer vorgegebenen oder vorgebbaren Zeitspanne mittels der Luftansaugvorrichtung 18 Umgebungsluft angesaugt und in das Gehäuse 12 gesaugt. Bei einem eventuellen Austritt von Schwergas im Bereich der Detektionsvorrichtung 10 umfasst die angesaugte Umgebungsluft auch das Schwergas; das Schwergas wird selbst angesaugt oder von der angesaugten Umgebungsluft mitgerissen. Das Schwergas gelangt daher mit dem Luftstrom durch das Gehäuse 12 auch zur Sensorik 20 und kann dort erfasst werden. Bei einer Detektion von Schwergas wird mittels der Sensorik 20 ein entsprechendes Signal erzeugt. Insoweit ist in den Figuren auf der Oberseite der Detektionsvorrichtung 10 ein Element zur Hindurchführung von Kabeln in das Innere des Gehäuses 12 und aus dem Innern des Gehäuses 12 gezeigt. Damit erfolgt die elektrische Versorgung der Detektionsvorrichtung 10, nämlich die Versorgung zumindest der Luftansaugvorrichtung 18 und der Sensorik 20. Damit erfolgt aber auch die Weiterleitung eventueller Signale von der Sensorik 20. Ein solches Signal kann an entfernter Stelle ausgewertet werden und führt dort zum Beispiel zu einer Alarmmeldung. Die Sensorik 20 umfasst zur Erkennung von Schwergas zum Beispiel ein optisches Messystem, insbesondere ein Messsystem mit einem sogenannten NDIR-Sensor, und/oder einen Halbleiter-Gassensor.

Bei der gezeigten Ausführungsform der Detektionsvorrichtung 10 besteht die Besonderheit darin, dass sich die Sensorik 20 in der Auffangvorrichtung 22 befindet. Hier gilt für den Luftstrom durch das Gehäuse 12, dass der Luftstrom durch die Auffangvorrichtung 22 hindurchströmt, indem dieser im Bereich der oder jeder Öffnung 32 in die Auffangvorrichtung 22 gelangt und diese seitlich an der Sensorik 20 vorbei sowie innen an dem umlaufenden Rand 26 vorbei den Bereich der Auffangvorrichtung 22 wieder verlässt. Von dem Luftstrom mitgerissenes Schwergas wird innerhalb der Auffangvorrichtung 22 zumindest zum Teil wieder in Richtung auf den Boden absinken und sinkt dort daher in Richtung auf den Boden der von der Auffangvorrichtung 22 umfassten Wanne 24 ab. Genau in diese Wanne 24 ist die Sensorik 20 gerichtet und kann dort somit mit dem angesaugten Luftstrom mitgerissenes Schwergas gut erfassen, zumal sich mitgerissenes Schwergas mit der Zeit in der Wanne 24 sammelt und sich die Schwergasmenge dort somit erhöht und schließlich jedenfalls eine Ansprechschwelle der Sensorik 20 überschreitet.

Abgesehen von dem Sammeleffekt der Wanne 24 der Auffangvorrichtung 22 haben die Wanne 24 und die Auffangvorrichtung 22 insgesamt noch den Effekt, dass sie die Sensorik schützen. Wenn zum Beispiel im Bereich der Detektionsvorrichtung 10 bei Reinigungstätigkeiten oder dergleichen ("Wischen") eine Wasser- oder Wasser-/Reinigungsmittelschicht auf dem Boden entsteht, könnte die Flüssigkeit in das Innere der Detektionsvorrichtung 10 gelangen und dort die Sensorik 20 benetzen und somit verunreinigen. Auch wenn die Flüssigkeit schließlich wieder verschwindet (abtrocknet), ist nach einer solchen Verunreinigung eine sichere Sensierung von Schwergas nicht mehr in jedem Fall gewährleistet.

Die Detektionsvorrichtung 10 kann zum Beispiel in einer Auffangwanne unter einer Kältemaschine platziert werden oder auf dem Boden eines Raums, in dem zumindest eine Kältemaschine betrieben wird, platziert werden. Jedenfalls wird die Detektionsvorrichtung 10 so platziert, dass sich deren Lufteinlass 14 so tief wie möglich befindet. Als Beispiel für einen Raum, in dem zumindest eine Kältemaschine betrieben wird, kommen ein Kellerraum oder dergleichen, aber zum Beispiel auch ein sogenannter Serverraum in Betracht. Beim Betrieb eines Servers oder mehrerer Server ist es üblich, dass der Raum, in dem sich dieser oder diese befindet bzw. befinden, mittels zumindest einer Kältemaschine klimatisiert wird.

Obwohl die Erfindung im Detail durch das Ausführungsbeispiel näher illustriert und beschrieben wurde, so ist die Erfindung nicht durch das oder die offenbarten Beispiele eingeschränkt und andere Variationen können der Fachperson hieraus abgeleitet werden, ohne den Schutzumfang der Erfindung zu verlassen.

Einzelne im Vordergrund stehende Aspekte der hier eingereichten Beschreibung lassen sich damit kurz wie folgt zusammenfassen: Angegeben wird eine Detektionsvorrichtung (Schwergasdetektionsvorrichtung) 10 mit einem Gehäuse 12 und mit einer Luftansaugvorrichtung 18, einer Sensorik 20 und einer Auffangvorrichtung 22 im Innern des Gehäuses 12 sowie mit zumindest einen Lufteinlass 14 in das Innere des Gehäuses 12, wobei sich die Sensorik 20 in einem Strömungsweg vom Lufteinlass 14 zur Luftansaugvorrichtung 18 befindet und in der Auffangvorrichtung 22 platziert ist. Die Detektionsvorrichtung 10 ist zur Detektion von Schwergas bestimmt und ist zur Detektion von Schwergas eingerichtet. Insoweit ist die hier vorgeschlagene Detektionsvorrichtung 10 eine Schwergasdetektionsvorrichtung 10. Mittels der Luftansaugvorrichtung 18 wird Umgebungsluft aus der Umgebung der Detektionsvorrichtung 10 in das Innere des Gehäuses 12 gefördert/gesaugt und von der Umgebungsluft ggf. mitgerissenes Schwergas gelangt in das Innere des Gehäuses 12 und zu der dort befindlichen Sensorik 20. Ein Austritt von Schwergas in der Umgebung der Detektionsvorrichtung 10 kann damit schnell und sicher auch bei nur geringen ausgetretenen Mengen erkannt werden. Im Innern des Gehäuses 12 befindet sich eine Auffangvorrichtung 22 zum Auffangen von ausgetretenem Schwergas und die Sensorik 20 ist in der Auffangvorrichtung 22 platziert oder zumindest in die Auffangvorrichtung 22 gerichtet. Dort sammelt sich in das Gehäuse 12 mitgerissenes Schwergas und aufgrund des Sammeleffekts der Auffangvorrichtung 22 ist bereits bei geringen Mengen von ausgetretenem Schwergas dessen Erkennung mittels der Detektionsvorrichtung 10 und der davon umfassten Sensorik 20 möglich. Die Auffangvorrichtung 22 ist in diesem Sinne eine Schwergasauffangvorrichtung 22 oder eine Schwergassammelvorrichtung. Die von der Auffangvorrichtung 22 umfasste Wanne 24 ist in diesem Sinne eine Schwergasauffangwanne 24 oder eine Schwergassammelwanne.

### Bezugszeichenliste

- 10: Detektionsvorrichtung, Schwergasdetektionsvorrichtung
- 12: Gehäuse
- 14: Lufteinlass
- 16: Luftauslass
- 18: Luftansaugvorrichtung
- 20: Sensorik
- 22: Auffangvorrichtung, Schwergasauffangvorrichtung
- 24: Wanne (der Auffangvorrichtung), Schwergasauffangwanne
- 26: Rand (der Auffangvorrichtung)
- 28: Filtertasche
- 30: Filterelement
- 32: Öffnung (in der Seitenwand der Wanne der Auffangvorrichtung)

## Patentansprüche

1. Schwergasdetektionsvorrichtung (10)
mit einem Gehäuse (12) und einer Sensorik (20) im Innern des Gehäuses (12),
mit zumindest einem Lufteinlass (14) in das Innere des Gehäuses (12) und einer Luftansaugvorrichtung (18) im Innern des Gehäuses (12),
wobei sich die Sensorik (20) in einem Strömungsweg vom Lufteinlass (14) zur Luftansaugvorrichtung (18) befindet,
**gekennzeichnet durch**
eine Auffangvorrichtung (22) im Innern des Gehäuses (12),
wobei die Sensorik (20) in der Auffangvorrichtung (22) platziert ist.

2. Schwergasdetektionsvorrichtung (10) nach Anspruch 1,
wobei die Auffangvorrichtung (22) eine Wanne (24) und einen umlaufenden Rand (26) aufweist und die Auffangvorrichtung (22) mit ihrem Rand (26) formschlüssig vom Gehäuse (12) aufgenommen wird und
wobei die Sensorik (20) in die Wanne (24) reicht.

3. Schwergasdetektionsvorrichtung (10) nach Anspruch 2,
wobei die Auffangvorrichtung (22) zumindest eine Öffnung (32) aufweist, durch welche mittels der Luftansaugvorrichtung (18) in der Umgebung der Detektionsvorrichtung (10) und durch den zumindest einen Lufteinlass (14) ansaugbare Umgebungsluft in die Auffangvorrichtung (22) und zu der dort befindlichen Sensorik (20) gelangt.

4. Schwergasdetektionsvorrichtung (10) nach einem der vorangehenden Ansprüche,
wobei sich der zumindest eine Lufteinlass (14) im Bereich einer Unterkante des Gehäuses (12) befindet.

5. Schwergasdetektionsvorrichtung (10) nach einem der vorangehenden Ansprüche,
mit zumindest einem Luftauslass (16) aus dem Innern des Gehäuses (12),
wobei die Sensorik (20) in einem Strömungsweg von dem oder jedem Lufteinlass (14) zu dem oder jedem Luftauslass platziert ist.

6. Schwergasdetektionsvorrichtung (10) nach einem der vorangehenden Ansprüche,
mit einer Schmutzpartikelrückhaltevorrichtung im Strömungsweg von dem oder jedem Lufteinlass (14) zur Sensorik (20).

7. Detektionsvorrichtung (10) nach einem der vorangehenden Ansprüche,
wobei sich die Luftansaugvorrichtung (18) im Innern des Gehäuses (12) über der Sensorik (20) befindet.

8. Schwergasdetektionsvorrichtung (10) nach einem der vorangehenden Ansprüche,
wobei sich der oder jeder Luftauslass (16) im Innern des Gehäuses (12) über der Sensorik (20) befindet.

9. Schwergasdetektionsvorrichtung (10) nach einem der vorangehenden Ansprüche,
wobei das Gehäuse (12) eine Grundfläche und eine Höhe aufweist,
wobei die Höhe des Gehäuses (12) größer ist als jede Dimension der Grundfläche.
